# EUROPEAN PATENT SPECIFICATION

(11) **EP 2 624 882 B1**
(45) Date of publication and mention of the grant of the patent: **28.04.2021**
(21) Application number: 10768713.9
(22) Date of filing: 06.10.2010
(51) Int. Cl.: A61M 5/20, A61M 5/32

(54) **LOCKING AND RETAINING MECHANISM FOR THE NEEDLE GUARD SLEEVE OF AN INJECTION DEVICE**
BLOCKIER- UND RÜCKHALTEMECHANISMUS FÜR DIE NADELSCHUTZHÜLLE EINER INJEKTIONSNADEL
MÉCANISME DE VERROUILLAGE ET DE RETENUE POUR LE PROTECTEUR D'AIGUILLE D'UN DISPOSITIF D'INJECTION

(43) Date of publication of application: 14.08.2013
(73) Proprietor: Ypsomed AG, 3401 Burgdorf (CH)
(72) Inventor: PEYER, Dominique, 3053 Münchenbuchsee (CH); STREIT, Ursina, 3322 Schönbühl (CH); HIRSCHEL, Jürg, 5000 Aarau (CH); MOSER, Ulrich, 3412 Heimiswil (CH); KAUFMANN, Nadine, 3400 Burgdorf (CH)
(74) Representative: Kleiner, Stefan
(86) International application number: PCT/EP2010/064952
(87) International publication number: WO 2012/045350

(56) References cited:
- EP-A1- 1 932 558
- WO-A1-2010/018411
- DE-A1-102007 013 837
- GB-A- 2 463 034
- US-A- 6 099 503
- US-A1- 2003 105 430

## Description

The invention relates to an injection device which has a mechanism for automatically discharging what is in particular a liquid product or medicament. The medicament might be a growth hormone or an interferon, to name but a couple of examples. The injection device may be designed such that an injection is administered manually by the needle. Alternatively, the injection device may comprise a mechanism for having a needle penetrate automatically. The invention in particular relates to an injection device according to the preamble of claim 1.

An injection device is known from DE 10 2007 013 837 A1, for example, which has a needle guard at its distal end and an activation mechanism at its proximal end which can be rotated relative to the needle guard. The activation mechanism can be rotated from a non-activated position into an activated position. When the activation mechanism is in its non-activated position, the needle guard can not be pushed back relative to a housing of the injection device because the activation mechanism has an axial stop against which a snapper element pushed in the proximal direction with the needle guard is moved. When the activation mechanism is in its activated position, this stop is moved away, thereby enabling the needle guard to be moved in the proximal direction. After administering, the needle guard sleeve can be pushed back into the housing, which may be prevented by rotating the activation mechanism back into its non-activated position.

WO 02/04053 A1 discloses a needle guard which can be rotated relative to a housing into different rotation positions. The needle guard has a protuberance which is aligned with stops disposed at different heights depending on the rotation position of the needle guard. Depending on the stop with which the protuberance is aligned, the needle guard can be pushed back by different degrees or can be prevented from being pushed back at all. After administering, the needle guard must be rotated so that it is in alignment with a specific stop, because otherwise it would be possible to push it back and its function as a needle guard could be fulfilled to a limited degree only. If the person using the device forgets to rotate the needle guard after the administering operation, there is a risk of injury by the needle.

Accordingly, a problem to be solved by the invention is to provide an injection device which reduces the risk of injury even better.

This problem is solved by the injection device according to claim 1. Advantageous embodiments are defined in the dependent claims.

The invention is based on the injection device outlined above, which has a mechanism for automatically discharging a product. The injection device comprises a preferably sleeve-shaped and cylindrical housing. The injection device also comprises a needle guard at the distal end of the injection device or housing, i.e. at the end at which also the injection needle is disposed. The needle guard is displaceable relative to the housing and is preferably not rotatable relative to the housing. For example, the needle guard may be guided along the housing. As will be described herein, however, the ability of the needle guard to move is restricted or can be restricted depending on the operating mode of the injection device. The needle guard may be moved into the housing from its initial position into a proximal end position.

In the initial position, the needle guard preferably assumes a position in which it laterally surrounds the needle tip. In particular, the needle tip is located proximally of the distal end of the needle guard. The needle guard is preferably sleeve-shaped. The needle guard may be disposed outside or preferably inside the housing. In the initial position, the needle guard preferably extends distally beyond the distal end of the housing.

In the proximal end position, the needle guard is pushed back relative to the housing, wherein the needle guard protrudes beyond the distal end of the housing to a lesser degree than it does when the needle guard is in the initial position or sits flush with the housing. For example, the needle protrudes or extends beyond the distal end of the injection device, in particular of the needle guard, when the needle guard is in its proximal end position.

The injection device may be adapted so that when the needle guard is moved from its initial position into the proximal end position, the mechanism for automatically discharging the product is indirectly or directly released. In the case of a direct release, there is no intermediate step. In the case of an indirect release, an intermediate step may be executed. For example, the injection device may be adapted so that when the needle guard is moved into its proximal end position, a mechanism for automatic penetration of the needle can be triggered. This mechanism may be coupled with the mechanism for automatically discharging so that during or at the end of the penetration, the mechanism for automatically discharging the product can be triggered.

The injection device further comprises an activation mechanism, which can be moved, in particular rotated, from a non-activated position into an activated position. The activation mechanism is preferably disposed at the proximal end of the injection device or housing, and in particular can be rotated relative to the housing but is not able to move axially. The activation mechanism may preferably be a rotating button. For example, the housing may have a first mark and a second mark, and the activation mechanism has an indicator which points to the first mark in the non-activated position and to the second mark in the activated position. This offers the user a simple way of telling whether the device is activated or not activated.

When the activation mechanism is in the non-activated position, the needle guard is blocked from being able to move into the proximal end position. When the activation mechanism is in the activated position, on the other hand, the needle guard can be moved into the proximal end position. In the non-activated position, the needle is not able to protrude past the distal end of the needle guard. In the activated position, the needle projects beyond or extends beyond the distal end of the needle guard.

The injection device has at least one locking means, at least one of which is a spring-operated locking means, and at least one transmission means, wherein the at least one transmission means moves the spring-biased locking means from a first position or locked position into a second position as the activation mechanism is moved into the activated position, thereby deflecting and tensing it. When the activation mechanism is in the activated position, the needle guard is released so that it can be pushed from the initial position into the proximal end position as the locking means is moved out of the first position or blocking position. The locking and transmission means may be part of a transmission mechanism, wherein the transmission means slides off the locking means, as a result of which the locking means moves transversely to the movement of the transmission means. In particular, the transmission means and locking means 2. are designed so that the transmission means pushes the locking means out of its first position or blocking position. The transmission and locking means may have surfaces or edges which slide on one another and which are disposed according to the principle of an inclined plane which slides off an edge or another inclined plane. Due to the fact that in accordance with the invention the locking means which is disposed in a resilient manner is moved out of its first position or blocking position and tensed during activation, this locking means springs back into a position in which it blocks the needle guard preferably without the need for any user interaction. In the position into which it springs back, the resiliently arranged locking means may be disposed in front of or flush with a stop, in particular a second stop, against which it is pushed in an attempt to move the needle guard in the proximal direction relative to the housing. The device is safer as a result of the invention because when the activation mechanism is activated, the user already assures that the needle guard will be automatically blocked, whereby the user no longer needs to turn the activation mechanism into a non-activated position and can no longer forget to do this. This also avoids stored in a spring in order to perform a penetration with a needle and/or for discharging the product being deducted for tensing the locking means.

Since the locking means is arranged resiliently, it can be moved by the transmission means against the spring force out of the first position or blocking position. To obtain this resilient arrangement, a spring may be provided, such as a leaf or coil spring, for example, or preferably a resilient arm, which is disposed for example one of the activation mechanism and the needle guard. Each locking means may be formed on a resilient arm. Due to the resilient arrangement of the locking means and due to the fact that the locking means is moved by the transmission means against the spring force when the activation mechanism is moved from the non-activated position into the activated position, i.e. the spring element is pre-biased, the locking means can be moved into a blocking engagement, in particular without the user having to perform additional operating steps.

The device may have a first locking means, by means of which the needle guard sleeve can be blocked to prevent it from being moved back out of the initial position into the proximal end position, and a second, resiliently arranged locking means by means of which the needle guard sleeve can be blocked to prevent it from being pushed back out of the needle-guarding position into the proximal end position.

In its first position, the first locking means may be positioned in front of or flush with a first stop, whereby the movement of the needle guard out of the initial position in the proximal direction or into the proximal end position is blocked. In this position, the first locking means may be disposed in particular distally of the first stop. When attempting to move the needle guard into its proximal end position, the first locking means is pushed against the first stop.

When the needle guard is in the needle-guarding position, the second locking means may be disposed in front of or flush with a second stop, whereby the movement of the needle guard out of the needle-guarding position in the proximal direction or into the proximal end position is blocked. In this position, the second locking means may be disposed in particular distally of the second stop. When attempting to move the needle guard into its proximal end position, the second locking means is pushed against the second stop.

When the activation mechanism is in the non-activated position, the first locking means can be positioned in its first position distally of and flush with the first stop. By rotating the activation mechanism, the first stop and the first lock element can be moved out of alignment and/or the flush arrangement. For example, the first stop can be rotated out of the alignment and/or flush arrangement and relative to the first locking means, wherein it is preferable that the first locking means is not deflected radially. Alternatively, the first locking means can be deflected and optionally tensed, in particular by a first transmission means, in particular in the radial direction away from or towards the longitudinal axis of the injection device. Due to the fact that the first stop and the first locking means are no longer in alignment and/or flush, the needle guard can be moved into the proximal end position or/and the first locking means can be moved in the proximal direction past the first stop.

Alternatively or additionally, by rotation of the activation mechanism, the second locking means can be deflected outwards by a second transmission means in particular, from its first position into its second position, in particular in the radial direction away from or towards the longitudinal axis of the injection device, and tensed. As the needle guard moves into its proximal end position, the second locking means slides along a slip surface and preferably remains deflected. As the needle guard is moved out of the proximal end position into the needle-guarding position or at the end of this movement, the second locking means is moved past in particular the second stop, during which the outwardly deflected and tensed second locking means snaps or springs into a position in front of the second stop in which it sits flush with the second stop, in particular in the radial direction away from or towards the longitudinal axis of the injection device, i.e. in the direction opposite to the one in which it was deflected and tensed.

In preferred embodiments, the resiliently arranged locking means may block the needle guard sleeve to prevent it from both moving back out of the initial position into the proximal end position and from moving out of the position guarding the needle into the proximal end position.

In its first position, the locking means may be positioned in front of or flush with a first stop, as a result of which the needle guard is blocked from moving out of the initial position in the proximal direction or into the proximal end position. In this position, the locking means may be disposed in particular distally of the first stop. When attempting to move the needle guard into its proximal end position, the locking means is pushed against the first stop.

When the needle guard is in the needle-guarding position , the locking means may be positioned in front of or flush with a second stop, thereby blocking the needle guard to prevent it from being moved out of the needle-guarding position in the proximal direction or into the proximal end position. In this position, the locking means may be disposed in particular distally of the second stop. When attempting to move the needle guard into its proximal end position, the locking means is pushed against the second stop.

When the activation mechanism is in the non-activated position, the locking means may be positioned in its first position distally and flush with the first stop. By rotating the activation mechanism, the first stop and the lock element can be moved out of the flush arrangement. Preferably, the locking means can be deflected by the transmission means from its first position into its second position and tensed, in particular in the radial direction away from or towards the longitudinal axis of the injection device. Due to the fact that the first stop and the locking means are no longer flush, the needle guard can be moved into the proximal end position or/and the locking means can be moved in the proximal direction past the first stop.

During the movement of the needle guard is moved into its proximal end position, the locking means can slide along a slip surface and preferably remains deflected. As the needle guard is moved from the proximal end position into the needle-guarding position or at the end of this movement, the locking means is moved in particular past the second stop, as a result of which the deflected and tensed locking means snaps or springs into a position in front of the second stop, in which it is flush with the second stop. The locking means snaps or springs in particular in the radial direction, away from or towards the longitudinal axis of the injection device, i.e. in the direction opposite the one in which it was deflected and tensed.

Preferably, the needle guard can be pushed out of its proximal end position over the needle projecting out from the distal end into a needle-guarding position. In the needle-guarding position, the needle, in particular the needle tip, is at least laterally surrounded by the needle guard. In particular, the distal end of the needle guard extends beyond the distal end of the needle, i.e. the needle tip, in the needle-guarding position. The needle tip therefore no longer extends beyond the needle guard when the latter is disposed in its needle-guarding position. This reliably prevents pricking.

Generally speaking, it is preferable if the first stop is disposed in an axial position different from the axial position of the second stop by reference to the longitudinal axis of the injection device, in particular disposed proximally with respect to the second stop. Alternatively or additionally, the first locking means and the second locking means may be disposed in different axial positions with respect to the longitudinal axis. Due to at least one of these features, the needle guard sleeve projects from the housing by a different distance in its needle-guarding position than when in its initial position. More preferably, the needle guard sleeve projects farther from the housing in its needle-guarding position than it does in its initial position.

During the movement of the needle guard into the needle-guarding position or in the needle-guarding position, the locking means, which is for example biased by the transmission means, can be moved into or in particular snap into a blocking engagement. The snapping action is made possible by the resilient arrangement. Generally speaking, the needle guard can be blocking in the needle-guarding position to prevent it from being moved back, i.e. from moving in the proximal direction relative to the housing. The locking means can be moved, in particular is able to spring, into a blocking engagement with the housing or the activation means or some other element at least fixedly disposed on the housing.

In preferred embodiments, the transmission means is designed so that it is able to move the locking means in the radial direction or alternatively transversely to the radial direction, in particular in the circumferential direction, from the first position, in particular during the movement of the activation mechanism into the activated position. The locking means may be moved out of the first position radially outwards or preferably radially inwards, i.e. towards the central axis of the device. Having been moved out of the first position into the second position, the locking means may lie against a surface, in particular an inwardly facing surface, the surface extending along the longitudinal direction of the injection device, and the locking means sliding along the surface during the movement of the needle guard out of its initial position into its proximal end position, and preferably during its movement out of the proximal end position in the direction towards the needle-guarding position. The surface may therefore be also described as a slip surface or guide surface and may be formed by, for example, the activation mechanism.

The first and the second stop are preferably axially fixed but rotatable relative to the housing. The first stop and the second stop may be formed on the same part, in particular on the activation mechanism, such as the transmission means. Preferably, the first and the second stop and the transmission means can be moved jointly, in such that the first and second stop are stationary relative to, for example, the transmission means. The first and second stop and the transmission means may therefore constitute a single piece and/or may be integrally formed and may be disposed in particular on the rotating button. When attempting to move the needle guard in the proximal direction, this will not be successful or there will be a movement by only a small amount, in particular a slackness, if the at least one locking means is in the blocking position, i.e. in front of and flush with the first or second stop. When the locking means is outside of the blocking position or/and when the activation mechanism is in the activated position, the locking means can be moved past the first stop in the axial direction.

The second stop may be formed, for example, by the housing, the activation means ore some other element which is stationary with respect to the housing, at least in terms of axial movement. The second stop is preferably formed on the part on which the transmission means is formed. For example, the second stop, the first stop and the transmission means may constitute a single part and/or be formed integrally. The second stop may bound the slip or guide surface mentioned above in the distal direction. As a result, the at least one locking means is able to slide along the slip or guide surface during the movement of the needle guard towards the needle-guarding position and finally snap or spring into the blocking engagement relative to the second stop, i.e. into a flush arrangement with the second stop.

In preferred embodiments, the activation mechanism may be operatively connected to the transmission means so that a movement, in particular a rotation, of the activation mechanism causes a movement, in particular a rotation, of the transmission means. The activation mechanism may be operatively connected to the transmission means, for example by means of an interconnected element or a transmission. The transmission means is preferably integrally formed with the activation mechanism. The needle guard may be operatively connected to the at least one locking means so that a movement, in particular an axial movement, of the needle guard causes a movement, in particular an axial movement, of the at least one locking means. The needle guard may be operatively connected to the at least one locking means, for example by means of an interconnected element or transmission. The locking means is preferably integrally formed on the needle guard.

In alternative preferred embodiments, the activation mechanism may be operatively connected to the at least one locking means so that a movement, in particular a rotating movement, of the activation mechanism causes a movement, in particular a rotating movement, of the at least one locking means. The activation mechanism may be operatively connected to the at least one locking means by means of an interconnected element or transmission. The at least one locking means is preferably integrally formed on the activation mechanism. The needle guard may be operatively connected to the transmission means so that a movement, in particular an axial movement, of the needle guard causes a movement, in particular an axial movement, of the transmission means. The needle guard may be operatively connected to the transmission means by means of an interconnected element or transmission. The transmission means is preferably integrally formed on the needle guard.

In preferred embodiments, the needle guard may have a trigger mechanism, which can be moved into the housing or the proximal end position in order to trigger discharging of the product. Thereby, an energy-storing means, such as a spring or a gas generator, may release energy in order to discharge the product. Such a spring might be a gas pressure spring or a coil or helical spring, for example. Energy is preferably stored in the spring when the needle guard is in its initial position. The spring may for example store energy by being biased.

The injection device may also have a back-rotation lock, which prevents the activation mechanism from being turned back into the non-activated position once it has been moved into the activated position. For example, a first rotation-locking surface may be provided on the activation mechanism, in particular the rotating button. A second rotation-locking surface may be provided on the needle guard or a part secured to it, in particular in the region of the at least one stop. When attempting to turn the activation mechanism out of the activated position into the non-activated position, the first rotation-locking surface is pushed against the second rotation-locking surface, whereby any backward rotation is blocked. In particular, the second rotation-locking surface may be formed by a rib, a flank of the rib pointing in the circumferential direction constituting the second stop surface. The oppositely lying flank of the rib is preferably slant and designed so that the transmission means of the activation mechanism slides along the slant surface when the activation mechanism is turned into the activated position. As a result, the locking means is deflected out of the first position into the second position.

The rib on which the second rotation-locking surface is formed is preferably disposed perpendicular to an axial stop surface formed by the locking means. The locking means and rib may together form an L-shaped web. Alternatively, a U-shaped web would be possible.

The injection device may have an actuator or return spring, against the force of which the needle guard can be moved from its initial position into the proximal end position. Thereby, the spring can be tensed, i.e. energy can be stored in the spring. This energy may be used to move the needle guard towards the needle-guarding position. Accordingly, the needle guard can be moved relative to the housing by means of the actuator or return spring in the direction towards the needle-guarding position or into the needle-guarding position.

In preferred embodiments, the injection device is designed so that the injection needle is stationary relative to the housing, i.e. also when the needle is penetrating, and extends beyond the distal end of the housing when the needle guard is in the initial position. In its initial position, the needle guard may surround the needle, including the needle tip. Due to the fact that the needle guard can be moved into the proximal end position when the activation mechanism is in its activated position, the needle is able to project out from the distal end of the needle guard. The distal end of the needle guard is therefore pushed behind the needle tip. With this type of injection device, the user applies the force for penetration by the needle himself, i.e. pierces with the needle manually or by hand. The user grips the housing and pushes the injection device by the distal end of the needle guard against the desired injection site. As a result, the needle guard is pushed back and the needle moves out from the distal end of the injection device.

In alternative preferred embodiments, the injection device is designed so that, in order to have the needle penetrate, the injection needle can be moved relative to the housing from a proximal position, in which the needle including the needle tip is disposed inside the injection device, in the distal direction relative to the housing. As a result, the injection needle moves out of the distal end of the injection device so that the injection needle projects out from the distal end of the injection device, in particular by the desired injection depth of the injection needle. With this type of device, the penetration takes place automatically. The needle preferably latches so that it is axially secured to the housing in its injection position, preferably via intermediate elements, and in particular is non-releasably latched. For the sake of completeness, it should be pointed out that another option would be to enable the needle to be pulled out of its injection position back into the housing.

Generally speaking, the needle is or can be attached to a product container disposed in the injection device or in the housing. For example, the product container may be a carpule to which the needle can be attached. In preferred embodiments, the product container is a syringe, to which the needle is already non-detachably fitted. The needle and product container may be fitted so that they are axially fixed relative to the housing, i.e. also during the injection, or displaceable relative to the housing. The product container may be accommodated in a product container holder constituting for example an intermediate element which can be snapped onto the housing or another element that is stationary relative to the housing in order to prevent a movement of the product container and needle relative to the housing. In the case of an automatically penetrating needle, the product container may be displaceable jointly with the product container holder. In particular, a penetration spring may be provided, which pushes the needle, in particular together with the product container and preferably also with the product container holder, in order to move the needle, for example. This penetration spring is preferably biased, in particular when the needle guard is in the initial position. The penetration spring is part of the mechanism for penetrating automatically with the injection needle.

As an alternative to providing a separate penetration spring, the penetrating movement of the needle may be caused by the energy-storing means, which supplies the energy for discharging the product.

The invention has been described on the basis of various embodiments. Other preferred embodiments will be described below with reference to the drawings. The features disclosed thereby constitute the subject matter of the invention and additional features, both individually and in any combination of features. Of the drawings:
- Figure 1a: illustrates a first embodiment of an injection device proposed by the invention, with a locking means disposed in a blocking engagement,
- Figures 1b and 1c: show details from Figure 1a,
- Figure 2a: shows the injection device illustrated in Figure 1a but with the locking means out of the blocking engagement,
- Figures 2b and 2c: show details from Figure 2a,
- Figure 2d: is an illustration of a part of a needle guard and a part of a plunger rod,
- Figure 3a: shows the injection device illustrated in Figure 1a, with a needle guard in its proximal end position,
- Figure 3b: shows a detail from Figure 3a,
- Figure 4a: shows the injection device from Figure 1a, with the locking means in a blocking engagement,
- Figure 4b: shows a detail from Figure 4a,
- Figures 5a, 5b: are exploded views of an activation mechanism in different rotational positions relative to a needle guard according to a second embodiment of an injection device,
- Figures 6a, 6b: are sectional views of a second embodiment of an injection device with a cap fitted, Figure 6b showing a view rotated 90° about the longitudinal axis compared with Figure 6a,
- Figures 7a, 7b: are sectional views showing the injection device illustrated in Figures 6a, 6b with the cap removed, with a needle guard in its initial position and an activation mechanism in its non-activated position, Figure 7b showing a view rotated by 90° about the longitudinal axis compared with Figure 7a,
- Figures 8a, 8b: are sectional views showing the injection device illustrated in Figures 6a, 6b with the activation mechanism in an activated position, Figure 8b showing a view rotated by 90° about the longitudinal axis compared with Figure 8a,
- Figures 9a, 9b: are sectional views showing the injection device illustrated in Figures 6a, 6b with the needle guard in its proximal end position, Figure 9b showing a view rotated by 90° about the longitudinal axis compared with Figure 9a,
- Figures 10a, 10b: are sectional views showing the injection device illustrated in Figures 6a, 6b for which a penetration sequence has been triggered, Figure 10b showing a view rotated by 90° about the longitudinal axis compared with Figure 10a,
- Figures 11a, 11b: are sectional views showing the injection device illustrated in Figures 6a, 6b for which a discharging sequence has been triggered, Figure 11b showing a view rotated by 90° about the longitudinal axis compared with Figure 11a,
- Figures 12a, 12b: are sectional views showing the injection device illustrated in Figures 6a, 6b with the needle in its penetration position, Figure 12b showing a view rotated by 90° about the longitudinal axis compared with Figure 12a,
- Figures 13a, 13b: are sectional views showing the injection device illustrated in Figures 6a, 6b with a device for generating a clicking sound at the end of discharging which is activated, Figure 13b showing a view rotated by 90° about the longitudinal axis compared with Figure 13a,
- Figures 14a, 14b: are sectional views showing the injection device illustrated in Figures 6a, 6b where the device for generating a clicking sound has generated a clicking sound, Figure 14b showing a view rotated by 90° about the longitudinal axis compared with Figure 14a,
- Figures 15a, 15b: are sectional views showing the injection device illustrated in Figures 6a, 6b with the needle guard in the needle-guarding position.

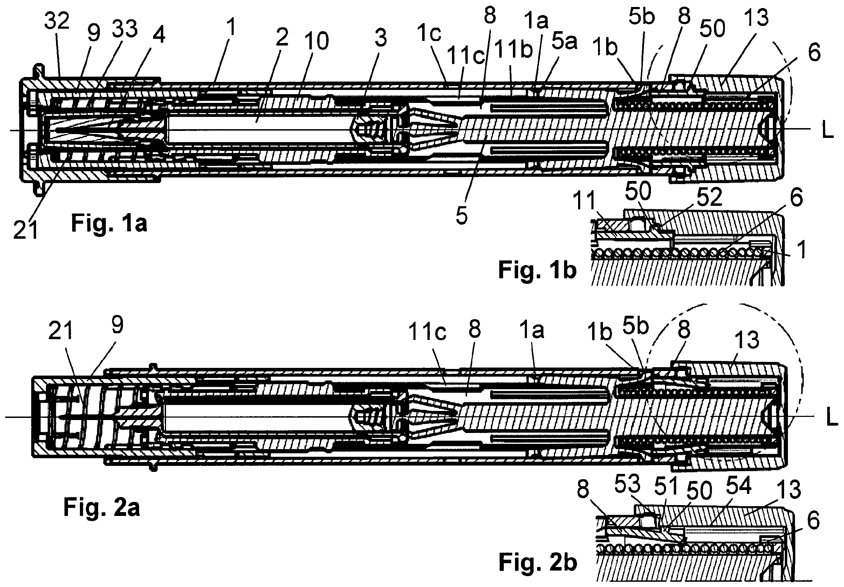

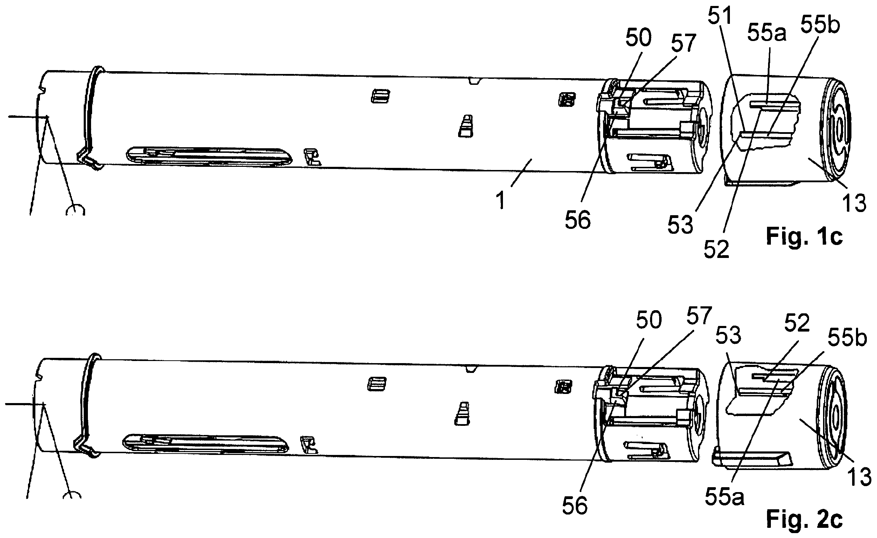

Figures 1a to 4b illustrate an injection device which is adapted to discharge automatically a product contained in a product container designed as a syringe 2. The penetration is performed manually, i.e. by hand. In Figure 1, the device is illustrated in the state in which it is delivered and comprises a cylindrical, sleeve-shaped housing 1, on the distal end of which a cap 32 is detachably fitted, and on the proximal end of which an activation mechanism in the form of a rotating button 13 is disposed. In the state of delivery, the rotating button 13 is in a non-activated position. The product container 2 is disposed in a sleeve-shaped product container holder 2 and is supported on an inwardly directed shoulder of the product container holder 10 by a shoulder disposed distally of the medicament portion of the product container 2. Accordingly, an outwardly extending flange at the proximal end of the product container 2, which may also be described as a finger flange, may be disposed at a distance from the product container holder 10, such that damage of the finger flange is avoided if the finger flange were pressed against the proximal end of the product container holder 10. The product container holder 10 is connected to the housing 1 so that it is not able to move axially, in particular by a snap-fit connection. An injection needle 4 is non-detachably connected to the product container 2 and may therefore be regarded as part of the product container 2. In the state of delivery, the injection needle 4 extends beyond the distal end of the housing 1. The injection needle 4 is covered by a needle guard cap 33, known as a rigid needle shield in the prior art. The needle guard cap 33 is supported on the attaching portion of the needle 4 on the product container 2. The cap 32 is designed so that the needle guard cap 33 is removed from the injection device when the cap 32 is removed from the injection device. Preferably, the cap 32 is removed from the injection device by pulling it off. The cap 32 has at least one engagement element, which engages with the needle guard cap 33 so that the needle guard cap 33 follows and/or is engaged at the same time as the cap 32 is pulled off. The at least one locating element may be a claw made from metal or an engaging cam made of plastic, for example.

The product container 2 comprises a plunger 3, which is able to move relative to the product container 2 in order to discharge the product. The plunger 3 can be moved by means of a plunger rod 5 disposed proximally of the plunger 3. Disposed between the plunger rod 5 and housing 1 is a driving spring 6 which is biased in the state of delivery and by means of which the plunger rod 5 can be driven in the discharging direction. The plunger rod 5 comprises at least one, preferably two locking elements 5a engaging with a locking stop 1a formed by the housing 1, in particular in the form of an inwardly pointing protuberance. The fact that the locking element 5a engages with the locking stop 1a prevents the driving spring 6 from releasing the energy stored in it to drive the plunger rod 5. The lock element 5a is disposed on a resilient arm, which is formed integrally with the plunger rod 5 and projects in the distal direction at the side of the plunger rod 5.

The injection device further comprises a needle guard in the form of a needle guard sleeve 9, which also serves as an operating sleeve for discharging the product. The needle guard sleeve 9 secured against rotation relative to the housing 1 but is able to move axially. In its initial state, the needle guard sleeve 9 extends beyond the distal end of the housing 1 and beyond the distal end of the needle 4. When the cap 32 and needle guard cap 33 are removed from the injection device (Figure 2a), the needle guard sleeve 9 prevents access to the needle 4. Disposed between the needle guard sleeve 9 and product container holder 10 is a spring 21, which may also be described as a return spring. The spring 21 is a helical spring which acts in as a compression spring. The spring 21 is tensed as the needle guard sleeve 9 is pushed back relative to the housing 1, i.e. during a movement of the needle guard sleeve 9 in the proximal direction. The tensed spring 21 is able to push the needle guard sleeve 9 out of a proximal position back in the distal direction.

The needle guard further comprises a switch sleeve 8 which can be moved together with the needle guard sleeve 9. For example, the switch sleeve 8 and needle guard sleeve 9 may be snap-fitted to one another so that they are axially fixed. In principle, it would also be possible for the switch sleeve 8 to be integrally formed with the needle guard sleeve 9, i.e. to constitute a single piece with the needle guard sleeve 9. The switch sleeve 8 has a support surface 11b, which is disposed axially at the level where the locking element 5a engages with the locking stop 1a when the needle guard sleeve 9 is in the initial state. The support surface 11b holds the locking element 5a in engagement with the locking stop 1a. Disposed distally of the support surface 11b is a recess 11c, which can be moved axially to the level of the locking element 5a or locking stop 1a.

The plunger rod 5 further comprises at least one snapper 5b, which is preferably disposed to the side of the plunger rod 5 and as an arm formed integrally with the plunger rod 5, the arm projecting in the proximal direction. In the state of delivery and when the needle guard 9, 8 is in the initial state, the snapper 5b engages with a recess 1b of the housing 1. The snapper 5b serves to generate a clicking sound when the plunger rod 5 has discharged the product contained in the product container 2.

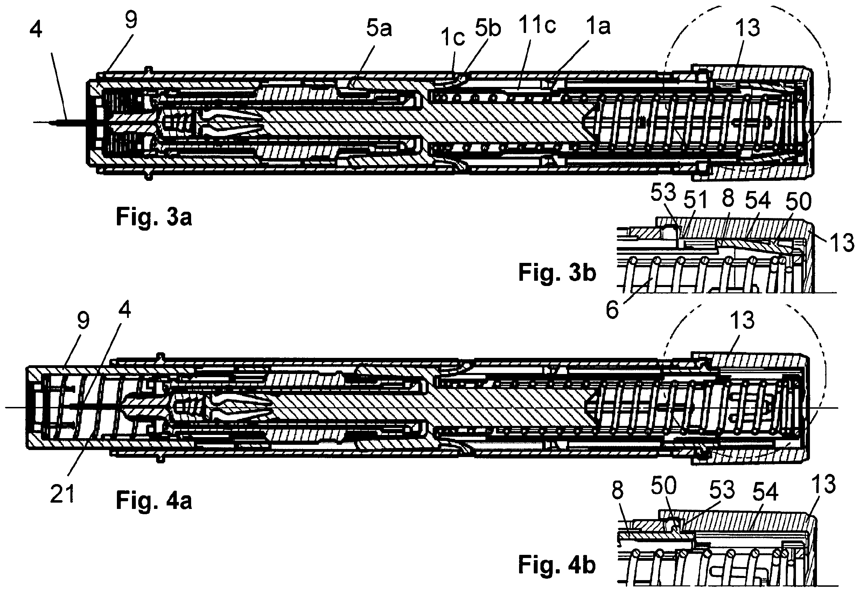

As best illustrated in Figures 1b and 1c, the activation mechanism 13 has on its internal circumference a first rib 55a, in particular extending parallel with the longitudinal axis L, a second rib 55b extending parallel with the longitudinal axis L and a first stop 52, which acts in the axial direction and is formed by the first rib 55a. An edge of the second rib 55b forms a transmission means 51. Alternatively, the transmission means 51 could be formed by a surface. The needle guard 8, 9, in particular the switch sleeve 8, has a locking means 50, which is resiliently connected via an arm to and integrally formed with the switch sleeve 8. The first stop 52 and locking means 50, and in particular also the central axis of the device, are disposed in one plane when the activation mechanism 13 is in the non-activated position. The locking means 50 is disposed in front of, i.e. flush with the first stop 52. Since the activation mechanism 13 is connected to the housing 1 so that it is not able to move axially but can rotate, a force applied to the needle guard sleeve 9 in the proximal direction causes the locking means 50 to be pressed against the stop 52. This prevents the needle guard sleeve 9 from being pushed back into a proximal end position, as illustrated in Figure 3a.

In order to administer the product, the user takes the cap 32 off and turns the activation mechanism 13 from the non-activated position (Figure 1a) into the activated position (Figure 2a, 2b). The transmission means 51 is able to slide on a slant surface 56 pointing in the circumferential direction on the arm and formed on the first stop 52 (Figure 1c, 2c). Thereby, the locking means 50 is deflected radially inwards by the transmission means 51, i.e. towards the longitudinal or central axis L, when the activation mechanism 13 is rotated from the non-activated position into the activated position as a result of which it moves out of the blocking position illustrated in Figure 1b and lies on a slip surface 54. When the activation mechanism 13 is in the activated position illustrated in Figure 2b, the locking means 50, longitudinal axis L and a second stop 53 and in particular also the second rib 55b are disposed in one plane. The slip surface 54 and the second stop 53 acting in the axial direction are formed by the second rib 55b. The slip surface 54 points inwards, i.e. towards the longitudinal axis L. In the position illustrated in Figure 2a, the needle guard sleeve 9 is no longer locked to prevent it from moving into the proximal end position.

In order to administer the product, the user holds the housing 1 and pushes the injection device by the distal end of the needle guard sleeve 9 against the desired injection site. As a result, the needle guard sleeve 9 is moved in the housing 1 into the proximal end position illustrated in Figure 3a so that the needle 4 extends out beyond the distal end of the needle guard sleeve 9 by a length corresponding to the desired injection depth. The activation mechanism 13 has the slip surface 54 extending in the longitudinal direction on its internal circumference on which the locking means 50 slides as the needle guard sleeve 9 moves.

As the needle guard sleeve 9 moves into the proximal end position, the support surface 1 1b is pushed far enough so that it is no longer axially at the position of the locked engagement between the locking element 5a and locking stop 1a. Instead, a recess 11c disposed distally of the support surface 11b is pushed axially so that it is on a level with the locked engagement. Since the support surface 1 1b is no securing that the locking element 5a is held in engagement with the locking stop 1a, the locking element 5a is pushed into the recess 11c, as a result of which the driving spring 6 is able to move the plunger rod 5 in the distal direction, i.e. in the discharging direction.

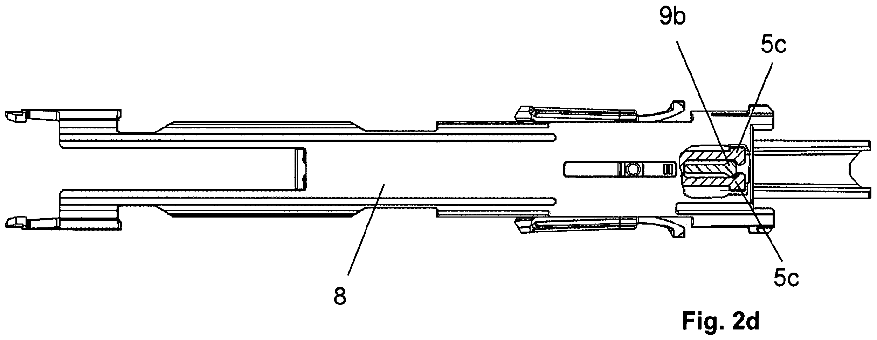

As may best be seen from Figure 2d, the switch sleeve 8 and plunger rod 5 are connected so that when the injection device is pressed onto the injection site, the pressing force increases and then abruptly decreases. To this end, the needle guard sleeve 9 has a cam 9b disposed between two lateral snappers, on the ends of which a cam 5c is formed respectively. A gap is formed between the cams 5c which is shorter than the width of the cam 9b. If the cam 9b is pushed against the cams 5c When attempting to push the needle guard sleeve 9 back into the housing 1, the cam 9b can initially not be pushed by the distance between the cams 5c so that the force needed to push the needle guard sleeve 9 must be increased, and when a threshold force is reached, the cams 5c move apart from one another, as a result of which the cam 9b can be pushed through the distance between the two cams 5c. As a result, the force applied to the needle guard 9 decreases abruptly. As a result, an initial resistance is reached which makes it more difficult to push the needle guard sleeve 9 back so that when the injection device is pressed against the injection site, a certain amount of pressing force must be applied in order to overcome the initial resistance, which drops abruptly as soon as the cam 9b is released from the engagement with the cams 5c, as a result of which the needle guard sleeve 9 is abruptly pushed back and the needle 4 abruptly effects the penetrating action.

At the end of the discharging sequence, i.e. of the movement of the plunger rod 5 in the discharging direction, the snapper 5b springs into a recess 1c formed in the housing 1, as a result of which a clicking sound indicates the end of the discharging sequence. This state is illustrated in Figure 3a. Figure 3b illustrates the position of the locking means 50 when the needle guard sleeve 9 is in its proximal end position.

After the end of the discharging sequence and after the user has optionally waited for 1 to 30 seconds until the medicament has been distributed in the tissue, the user pulls the injection device away from the injection site, as a result of which the spring 21 pushes the needle guard sleeve 9 relative to the housing 1 in the distal direction until the needle guard sleeve 9 extends beyond the distal end of the needle 4 and thus assumes its needle-guarding position in which access to the needle 4 is prevented. As may best be seen from Figure 4b, the locking means 50 slides along the slip surface 54 when the needle guard sleeve 9 is being moved into its needle-guarding position. During or preferably at the end of the movement into the needle-guarding position, the locking means 50 springs in front of a second stop 53 disposed distally of the slip surface 54. The second stop 53 acts as an axial stop against which the locking means 50 is pushed, if an attempt is made to move the needle guard 9 in the proximal direction out of its needle-guarding position. Consequently, the needle guard sleeve 9 is blocked against it from being pushed back, thereby preventing any risk of injury to the user.

Since the first stop and second stop are disposed in axially different positions, in particular by a distance corresponding to the width of the transmission surface 51, the needle guard sleeve 9 extends farther beyond the distal end of the housing 1 in its needle-guarding position than it does in its initial position in particular by this distance. By providing an according coloured marking on the external circumference of the needle guard sleeve 9 which only moves out of the housing 1 when the needle guard sleeve 9 is in its needle-guarding position, the user will immediately be able to tell whether the device has been used.

The injection device preferably has an back-rotation lock for the activation mechanism 13 for preventing back out of the activated position into the non-activated position. This back-rotation lock may be a rotation-locking surface 57, for example, which prevents the activation mechanism 13 from being rotated backwards due to the second rib 55b formed on the activation mechanism 13 being disposed in front of the rotation-locking surface 57 in the circumferential direction, so that in an attempt to turn the activation mechanism back into the non-activated position, the second rib 55b is pushed against the rotation-locking surface 57. The surfaces 56 und 57 are the flanks of a web forming one arm of an L-shape, the other arm of the L-shape being formed by the locking means 50.

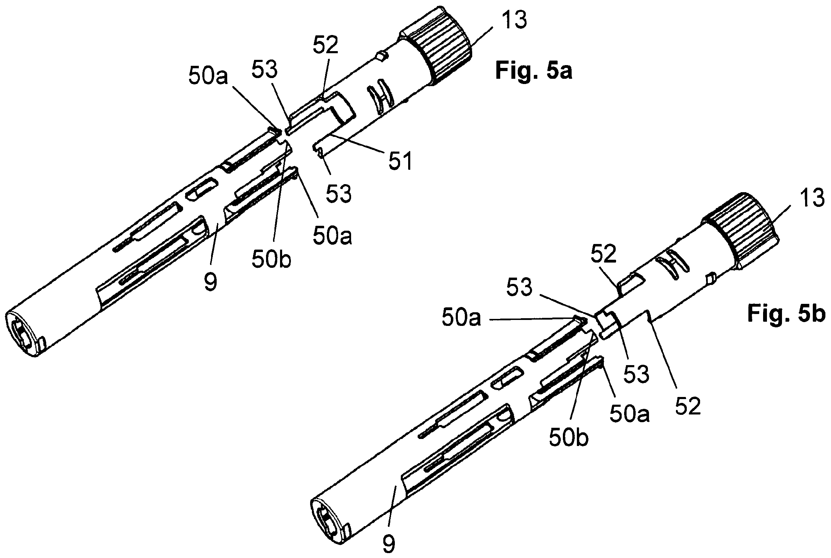

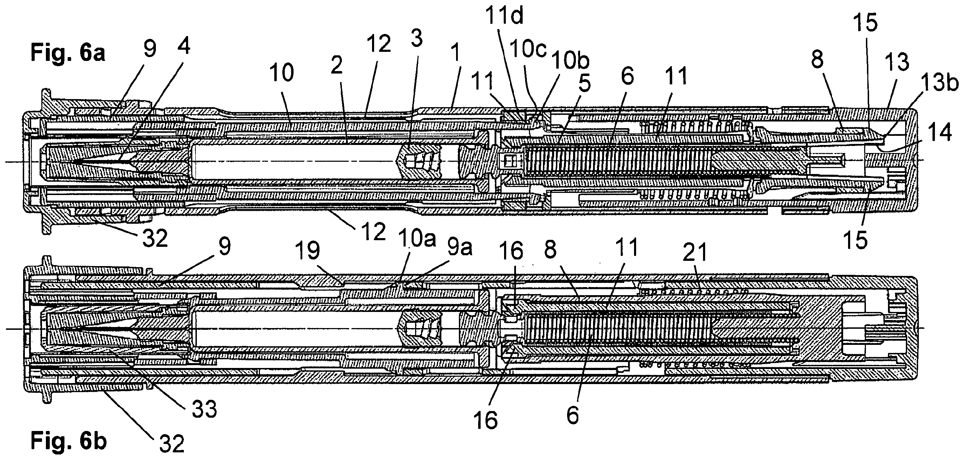

Figures 5a to 15b illustrate a further, second embodiment of an injection device according to the invention, the design of the locking means 50a and first stop 52 in Figure 5a, 5b being modified from the one in Figures 6a to 15b. The injection device is designed so that an automatic penetrating sequence takes place before the automatic discharging sequence. After the discharging sequence, the needle 4 remains in its penetrating position and a needle guard sleeve 9 is pushed over the distal end of the needle 4.

Figures 6a and 6b illustrate the injection device in a delivery state. The injection device has a cylindrical, sleeve-shaped housing 1, in which a product container 2 in the form of a syringe is accommodated so that it can be moved. Disposed on the distal end of the syringe is a non-detachable injection needle 4 for discharging a liquid product contained in the product container 2. The product container 2 has a displaceable plunger 3, the movement of which relative to the product container 2 and in the direction of the injection needle 4 causes the product to be discharged, for which reason this can be described as a discharging movement of the plunger 3. The product container 2 is accommodated in the device so that it is able to move in the distal direction, thereby enabling the injection needle 4 to be project out from the distal end of the injection device. This movement may be described as a penetration movement or penetrating sequence. The product container 2 is accommodated in a sleeve-shaped product container holder 10 so that it is not able to move axially and/or axially fixed or can move by only a certain clearance. The housing 1 has a viewing window 12 through which the user of the injection device is able to see through to the product container 2. The holder 10 surrounds the product container 2 in a sleeve-shaped arrangement so that it either by itself incorporates a viewing window to enable the container 2 to be seen or, as is the case in this example, is made from a transparent material.

As was the case with the first exemplary embodiment, a cap 32 is disposed at the distal end of the injection device, which can be pulled off the housing 1, and when it is being pulled off, it locates for example by means of a needle guard sleeve 33 covering the needle 4 and fitted on the syringe 2, takes the needle guard sleeve 33 with it during the pulling-off movement.

Disposed at the proximal end of the injection device or housing 1 is an activation mechanism 13 in the form of a rotating button which is not able to move axially and/or is axially fixed relative to the housing 1 and which can rotate relative to the housing 1. The activation element 13 can be rotated out of a non-activated position into an activated position.

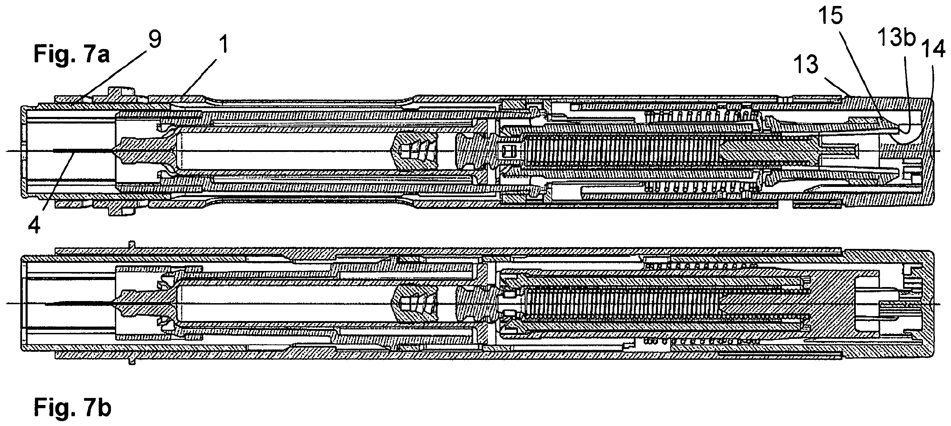

Disposed on the distal end of the injection device is a needle guard sleeve 9 which is disposed such that it can be moved relative to the housing 1 and which extends beyond the distal end of the housing. The needle guard sleeve 9 additionally serves as an operating sleeve and is part of a needle guard 8, 9. Proximally of the needle guard sleeve 9 a switch sleeve 8 is able to move relative to the housing 1, in particular is secured against rotation and can be moved axially. The switch sleeve 8 and needle guard sleeve 9 may apply pressing force one to the other, in particular latch with one another and mutually move one another as a result. In order for the needle guard sleeve 9 to not block the view onto the product container 2, the needle guard sleeve 9 also has a window in the region of the window 12. Alternatively, the needle guard sleeve 9 may be made from a transparent material. In its initial state (Figures 7a, 7b), the needle guard sleeve 9 is pushed by a return spring 21 distally beyond the distal end of the housing 1. The return spring 21, formed as a helical spring and acting as a compression spring, acts between the needle guard sleeve 9 and a functional sleeve 11. The return spring 21 is supported by its proximal end on the functional sleeve 11 and by its distal end on the needle guard sleeve 9. The return spring 21 is preferably biased with a low biasing force. The purpose of the return spring 21 is to apply a force to the switch sleeve 8 and needle guard sleeve 9, i.e. the needle guard 8, 9, acting in the distal direction so that the switch sleeve 8 is driven and the needle guard sleeve 9 is pushed in the distal direction, the product container holder 10 having an outwardly pointing projection 10a against which an axial stop 9a formed by the needle guard sleeve 9 is pushed by the spring 21. This prevents the spring 21 from pushing the needle guard sleeve 9 out of the housing 1 when the injection device is in the delivery state or the needle guard sleeve 9 is in its initial state.

At its proximal end, the product container holder 10 has a projection 10b disposed resiliently on an arm and in particular hook-shaped, which engages in a cut-out of the functional sleeve 11 when the needle guard 8, 9 is in the initial position. The cut-out has a stop 11d, against which the hook-shaped projection 10b abuts and/or is pushed by the spring 21 via the switch sleeve 8, needle guard sleeve 9 and product container holder 10. The functional sleeve 11 snap-fits onto the switch sleeve 8 by means of a snapper element 15. As a result, the spring 21 is not able to push the product container 2 and product container holder 10 in the distal direction into the position illustrated in Figures 6a, 6b.

The spring 21 is surrounded by the activation mechanism 13. The spring 21 surrounds the functional sleeve 11. The functional sleeve 11 surrounds a sleeve-shaped plunger rod 5 in which a biased driving spring 6 is disposed. When the device is in the initial state, the driving spring 6 is tensed so that the needle 4 and in particular the product container 2, product container holder 10 and functional sleeve 11 can be driven forwards for a subsequent operation of triggering the penetrating sequence and moving the plunger 3 so that it effects a discharging movement. The functional sleeve 11 has a locking element 16 on wich a shoulder directed radially inwards is formed, which shoulder in the initial state co-operates with a shoulder extending radially outwards at the distal end of the plunger rod 5 so that the plunger rod 5 is locked to prevent a movement relative to the functional sleeve 11. The locking element 16 is held in engagement with the plunger rod 5 by a surface of the switch sleeve 8 pointing radially inwards. The locking element 16 is preferably elastically connected to the functional sleeve 11 via a resilient arm and in particular is integral with it. The resilient arrangement may optionally be designed so that the locking element 16 has a tendency to move radially outwards, this being prevented by the surface of the switch sleeve 8 pointing radially inwards.

The functional sleeve 11 has at least one snapper element 15 at its proximal end, which snaps into the switch sleeve 8 in the initial state to prevent a movement of the functional sleeve 11. As a result, the tensed spring 6 is not yet able to relax and the functional sleeve 11 is still not able to move in the distal direction.

The activation mechanism 13 has an activation lock 14 which, when the injection device is in the switch states illustrated in Figures 6a, 6b, 7a, 7b, locates round the snapper element 15 so that the snapper element 15 is blocked or secured to prevent a movement out of the engagement with the switch sleeve 8. This advantageously prevents the injection device from being inadvertently triggered.

In order to use the device, the cap 32 and needle guard cap 33 (Figures 6a, 6b) are firstly removed from the injection device (Figures 7a, 7b). Figures 7a, 7b illustrate the needle guard cap 9 in its initial position in which it extends beyond the distal end of the needle 4. The activation mechanism 13 is in a non-activated position in which the activation lock 14 locates round the snapper element 15. When attempting to push the needle guard sleeve 9 into the housing 1, the snapper element 15 of the functional sleeve 11 abuts against an axial stop 13b of the activation element 13 (see Figure 7a), thereby preventing the needle guard sleeve 9 from being pushed back into its proximal end position. The snapper element 15 thus forms a first locking means and the axial stop 13b thus constitutes a first stop.

In the alternative arrangement illustrated in Figures 5a, 5b, a first locking means 50a of the needle guard sleeve 9 abuts against a first axial stop 52 of the activation element 13, the first axial stop 52 being assigned to the first locking means 50.

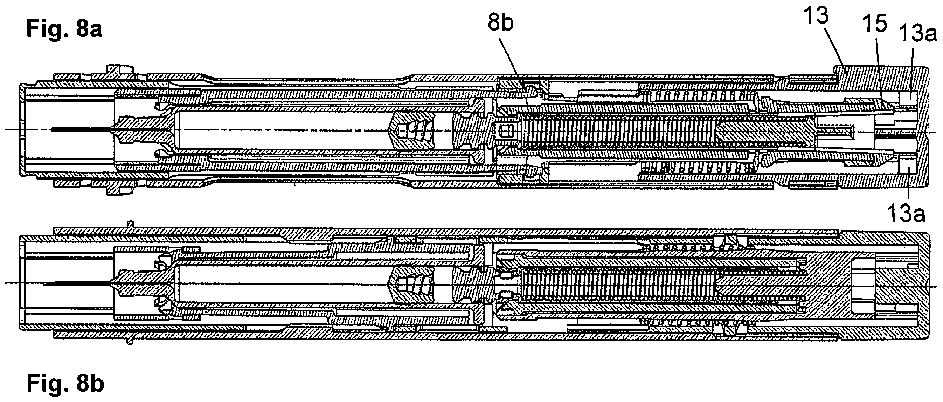

Figures 8a, 8b illustrate the activation mechanism 13 in its activated position. The injection device is activated or unlocked by a rotating movement of the activation mechanism 13, e.g. by 65° or 90°. In doing so, the axial stop 13b is rotated so that it moves out of the flush arrangement with the snapper element 15. In the modification illustrated in Figures 5a, 5b, the first locking means 50a is rotated out of the flush arrangement with the first stop 52. When the activation mechanism 13 is rotated, the snapper elements 15 are released so that they can effect an inwardly directed movement by the activation lock 14 being moved out of the engagement with the snapper elements 15, in particular being rotated. Consequently, there is enough space for the snapper elements 15 to be deflected inwards. The activation element 13 also has an activation cam 13a for the snapper element 15, which is moved by the rotating movement of the activation element 13 into axial alignment and/or flush arrangement with the snapper element 15, in particular is rotated. The snapper element 15 has proximally, and the activation cam 13a disposed proximally of it has distally a contour which can deflect the snapper element 15 radially inwards when the snapper element 15 moves out of engagement with the activation cam 13. In this example, a slant surface of the snapper element 15 slides on an edge formed by the activation cam 13 a.

Figure 5a illustrates the relative rotational position of the needle guard sleeve 9 and activation mechanism 13 in a non-activated position, but it is necessary to imagine the needle guard sleeve 9 and activation mechanism 13 pushed slightly farther together one inside the other. Irrespective of whether the first locking means and the first stop are formed as illustrated in Figures 5a, 5b or 6a to 15b, the needle guard sleeve 9 has a second locking means 50b for a second stop 53 formed on a sleeve-shaped portion of the activation mechanism 13 disposed in the housing 1. The needle guard sleeve 9 also has a slant surface pointing in the circumferential direction, which is able to co-operate with a transmission means 51 of the activation mechanism 13. When the activation mechanism is rotated out of the non-activated position into the activated position illustrated in Figure 5b or 8a, the transmission means 51 slides on the slant surface of the arm on which the second locking means 50b is disposed, thereby causing the second locking means 50b to be deflected inwards. The second stop 50b is out of alignment and/or out of flush arrangement with the second stop 53 in this position and lies against the internal circumference of the sleeve-shaped part of the activation mechanism 13. This enables the needle guard sleeve 9 to be moved in the proximal direction.

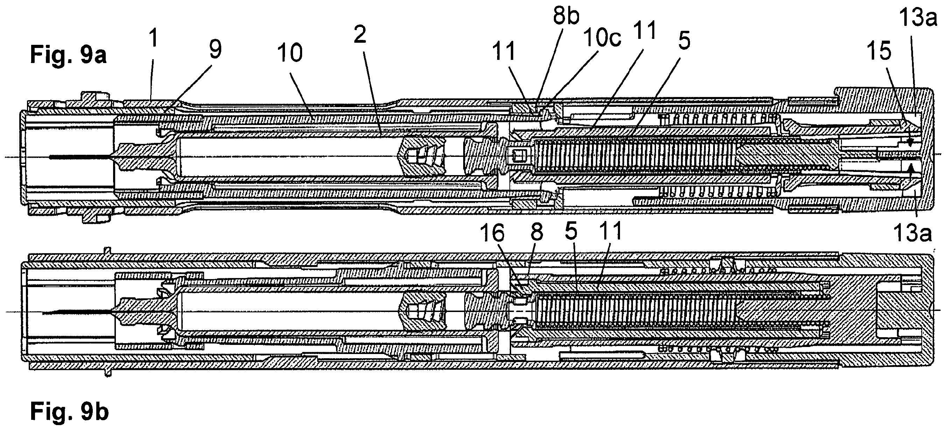

In order to trigger the injection device, the user presses the device by the distal end onto the injection site, which has preferably been disinfected beforehand. As a result, the needle guard sleeve 9 is pushed in the proximal direction relative to the housing 1, preferably until the distal end of the needle guard sleeve 9 is more or less flush with the distal end of the housing 1 (Figures 9a, 9b). As the needle guard sleeve 9 moves, the second locking means slides along the internal circumference of the sleeve-shaped part of the needle guard sleeve 13. Due to the movement of the needle guard sleeve 9, the switch sleeve 8 is driven in the proximal direction and the snapper elements 15 are released from the engagement with the switch sleeve 8 by means of the activation cams 13a, in particular being pushed radially inwards. Due to the movement of the needle guard sleeve 9 in the distal direction, the functional sleeve 11, product container holder 10 and product container 2 as well as the plunger rod 5 are also moved for as long as the snapper elements 15 remain snapped into the switch sleeve 8.

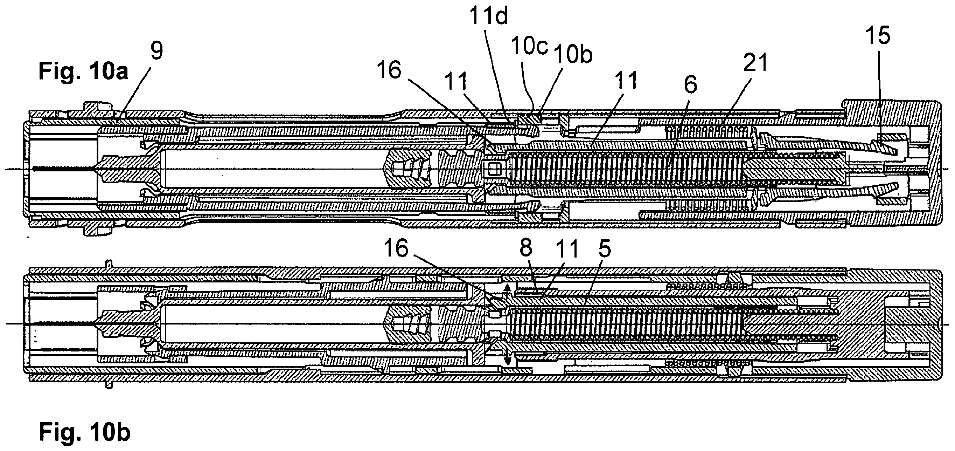

The release of the snappers 15 initiates a penetrating sequence. With the snappers 15 released, the energy stored in the spring 6 can be transmitted via the plunger rod 5 and locking element 16 to the functional sleeve 11so that the functional sleeve 11 moves in the distal direction relative to the housing 1 and switch sleeve 8. Thereby, the spring 21 is tensed and the spring 6 relaxed. The movement of the functional sleeve 11 relative to the switch sleeve 8 in the distal direction also causes the stop 11d to be moved in the distal direction. As a result, the product container holder 10 can be moved by the spring 21 relative to the housing 1 in the distal direction. Since the projection 10b has a transmission surface 10c inclined transversely to the driving direction, which is in alignment and/or flush arrangement with a transmission edge 11d formed on the switch sleeve 8a, the projection 10b is pushed radially inwards by the transmission edge 11d, the surface 10c now being in alignment and/or flush arrangement with the stop 11d (see Figure 10a)

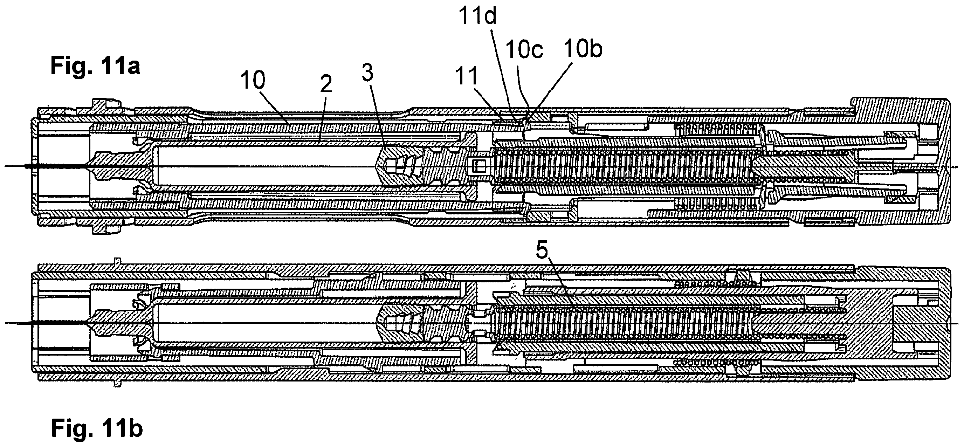

During the penetrating sequence, the functional sleeve 11 is moved relative to the switch sleeve 8 until the locking element 16 is disposed distally of the surface of the switch sleeve 8, which surface holds the locking element 16 in engagement with the plunger rod 5 (Figure 10b). Since the locking element 16 is now no longer held in engagement with the plunger rod, the locking elements 16 move, as indicated by the arrows in Figure 10b, out of the engagement with the plunger rod 5 so that the plunger rod 5 is released for movement in the distal direction relative to the functional sleeve 11. The plunger rod 5 is moved relative to the functional sleeve 11 and thus acts on the plunger 3, which drives the product container 2 with it due to the friction with the product container 2 and the inertia of the liquid contained in the product container 2 and therefore also drives the product container holder 10. Due to the driving action on the product container holder 10, the transmission surface 10c is pushed against the stop 11d on which the transmission surface 10c slides, causing the projection 10b to be deflected radially inwards (Figure 11a).

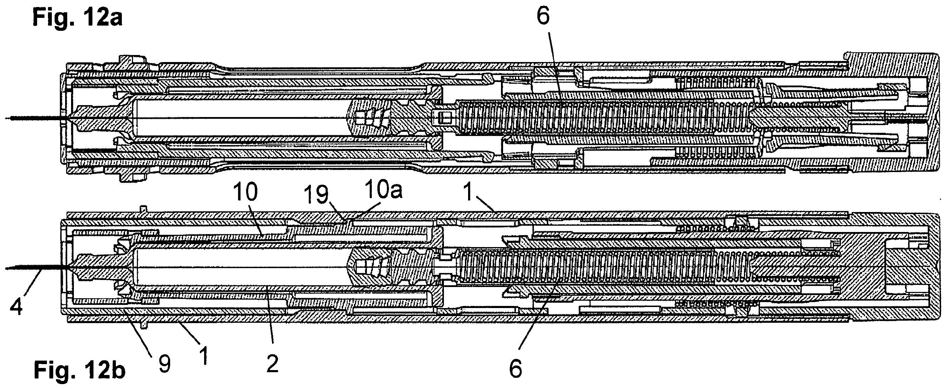

The spring 6 pushes the product container 2 and hence the needle 4 until the needle 4 extends out beyond the distal end of the housing 1 or needle guard sleeve 9 by the desired penetration depth. The end of the penetrating sequence and the length by which the needle 4 projects result from the positioning of an axial stop 19 against which the projection 10a of the product container 10 abuts at the end of the penetrating sequence. The stop 19 is formed by the housing 1. The end of the penetrating sequence is illustrated in Figures 12a and 12b.

Once the projection 10a has abutted against the stop 19, the spring 6 pushes the plunger 3 relative to the product container 2, causing the product contained in the product container 2 to be discharged via the needle 4.

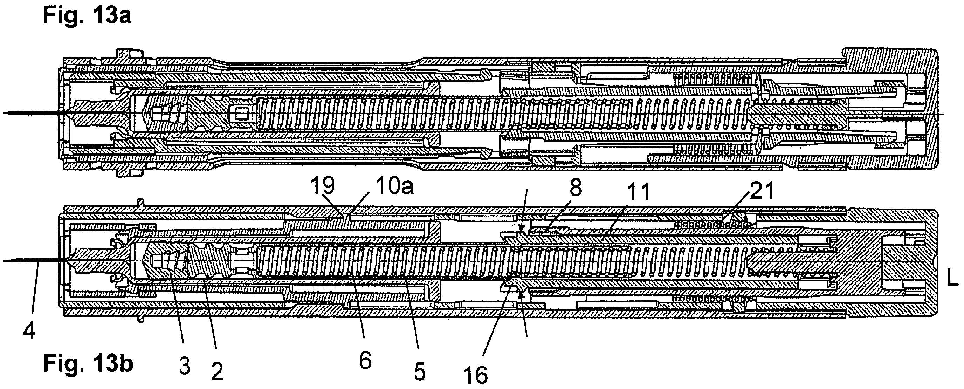

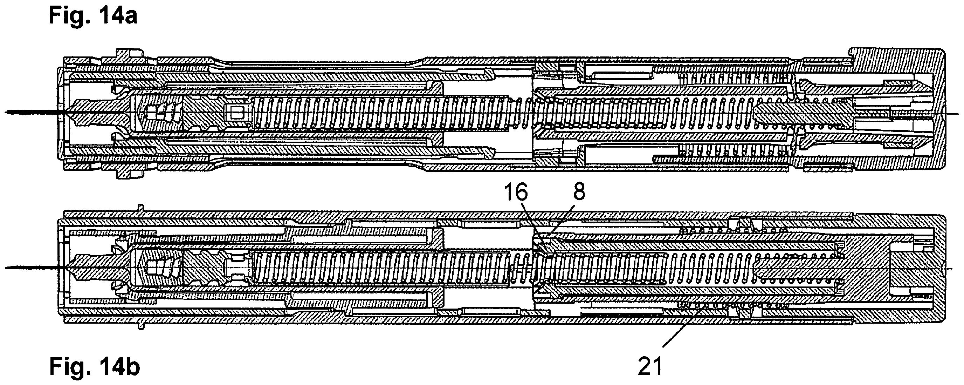

During the penetrating sequence and during the discharging sequence, the plunger rod 5 slides along the locking elements 16. As soon as the proximal end of the plunger rod 5 has moved past the locking elements 16, the snapper elements 15 mentioned above, which are preferably biased inwards, snap inwards, i.e. towards the longitudinal axis L, as indicated by the arrows in Figure 13b. The switch sleeve 8 has a recess at its proximal end for each of the locking elements 16, into which the locking elements 16 can be axially moved as they snap back towards the longitudinal axis L. Due to the energy stored in it, the spring 21 is able to move the functional sleeve 11 in the proximal direction relative to the switch sleeve 8, as a result of which the locking element 16 moves into the recess of the switch sleeve 8 provided for it until the locking element 16 axially abuts against an end of the recess (Figures 14a, 14b). As a result of this abutting, a clicking sound is generated, which provides an acoustic or/and tactile indication to the user of the device that the product has been completely discharged.

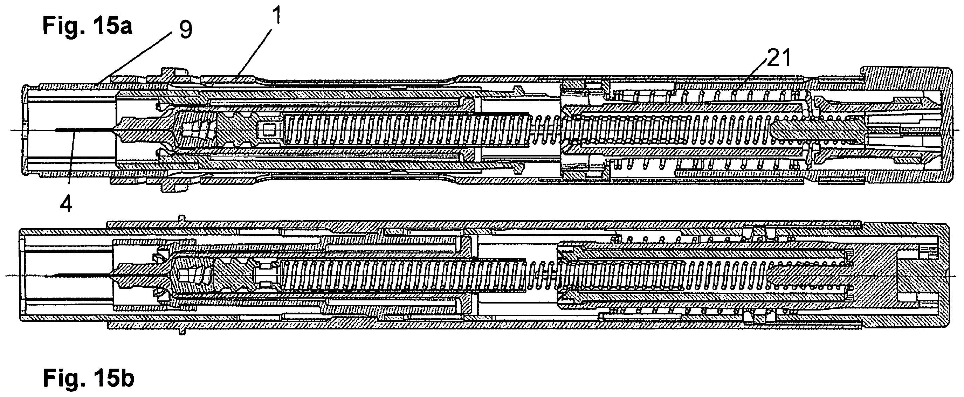

The user then takes the device away from the injection site, which causes the spring 21 to move the needle guard sleeve 9 in the distal direction relative to the housing 1. The needle guard sleeve 9 is moved until it has assumed its needle-guarding position, i.e. it extends beyond the distal end of the needle 4 and therefore protects the needle 4 from access (Figures 15a, 15b). In the needle-guarding position, a stop 9a formed by the needle guard sleeve 9 abuts axially against the projection 10a.

At the end of the movement of the needle guard sleeve 9 in the distal direction, the second locking means 50b is into a position in which it is disposed distally of the second stop 53, as a result of which the resiliently arranged locking means 50b springs outwards and thus sits in alignment and/or flush with the second stop 53. The needle guard sleeve 9 can therefore no longer be moved in the proximal direction. Due to the mutually engaging recesses on the second locking means 50 and the second stop 53, the activation mechanism is locked against rotational movement relative to the needle guard sleeve 9 back into a non-activated position.

**List of reference numbers**

| | | | |
|---|---|---|---|
| 1 | Housing | 11b | Support surface |
| 1a | Locking stop | 11c | Recess |
| 1b | Recess | 11d | Stop |
| 2 | Product container | 12 | Viewing window |
| 3 | Plunger | 13 | Activation mechanism |
| 4 | Injection needle | 13a | Activation cam |
| 5 | Plunger rod | 13b | Stop |
| 5a | Lock element | 14 | Activation lock |
| 5b | Snapper | 15 | Snapper element |
| 5c | Cam | 16 | Locking element |
| 6 | Driving spring | 19 | Axial stop |
| 8 | Switch sleeve | 21 | Return spring |
| 8a | Socket | 32 | Cap |
| 8b | Transmission edge | 33 | Needle guard cap/RNS |
| 9 | Operating sleeve/needle guard sleeve | 50 | Locking means |
| 9a | Stop | 50a | First locking means |
| 9b | Cam | 50b | Second locking means |
| 10 | Product container holder | 51 | Transmission means/transmission edge |
| 10a | Proj ection | | |
| 10b | Hook | 52 | First stop |
| 10c | Transmission surface | 53 | Second stop |
| 11 | Functional sleeve | 54 | Slip surface |
| 11a | Collar | 55a | First rib |
| | | 55b | Second rib |
| | | 56 | Surface |
| | | 57 | Rotation-locking surface |
| | | L | Longitudinal axis |

## Claims

1. Injection device having a mechanism for automatically discharging a product through an injection needle (4), further comprising:
a) a housing (1),
b) a needle guard (9) at the distal end of the injection device, which needle guard (9) can be moved from its initial position into a proximal end position into the housing (1) and from the proximal end position into a needle-guarding position in which the needle guard (9) surrounds the injection needle (4) projecting out from a distal end of the housing (1),
c) an activation mechanism (13) which can be moved from a non-activated position into an activated position, and when the activation mechanism (13) is in the non-activated position the needle guard (9) is blocked in its ability to be moved into the proximal end position and when the activation mechanism (13) is in the activated position the needle guard (9) can be moved into the proximal end position,
**characterized by**
d) a resiliently arranged locking means (50) or second locking means (50b) for blocking the needle guard (9) in the needle guarding position and a transmission means (51) which tenses and deflects the locking means (50) or the second locking means(50b) into a second position during the movement of the activation mechanism (13) into the activated position.

2. Injection device according to claim 1, **characterized in that** the locking means (50) or a first locking means (50a) is positioned in a first position in front of a first stop (52), as a result of which the movement of the needle guard (9) from the initial position in the proximal direction or into the proximal end position is blocked.

3. Injection device according to one of the preceding claims, **characterized in that** the locking means (50) or the second locking means (50b) is positioned in front of a second stop (53) when the needle guard (9) is in the needle-guarding position, as a result of which the movement of the needle guard (9) from the needle-guarding position in the proximal direction or into the proximal end position is blocked.

4. Injection device according to one of the preceding claims, **characterized in that** the transmission means (51) is adapted so that it moves the locking means (50) or the second locking means (50b) in the radial direction or transversely to the radial direction, in particular in the circumferential direction, out of its first position when the activation mechanism (13) is moved into the activated position.

5. Injection device according to one of the preceding claims, **characterized in that** the activation mechanism (13) is operatively connected to the transmission means (51) so that a movement, in particular a rotation, of the activation mechanism (13), causes a movement, in particular a rotation, of the transmission means (51), and that the needle guard (9) is operatively connected to the at least one locking means (50; 50a, 50b) so that a movement, in particular a longitudinal movement, of the needle guard (9) causes a movement, in particular a longitudinal movement, of the at least one locking means (50; 50a, 50b).

6. Injection device according to one of claims 1 to 4, **characterized in that** the activation mechanism (13) is operatively connected to the at least one locking means (50; 50a, 50b) so that a movement, in particular a rotation, of the activation mechanism (13) causes a movement, in particular a rotation, of the at least one locking means (50; 50a, 50b), and the needle guard (9) is operatively connected to the at least one transmission means (51; 5 1a, 51b) so that a movement, in particular a longitudinal movement, of the needle guard (9) causes a movement, in particular a longitudinal movement, of the at least one transmission means (51; 51a, 51b).

7. Injection device according to one of the preceding claims, **characterized in that**, the locking means (50) or second locking means (50b) can, by rotation of the activation mechanism (13), be deflected and tensed by the transmission means (51) into the second position, wherein the needle guard (9) can be moved into the proximal end position and the locking means (50) or second locking means (50b) can be moved in the proximal direction, and wherein during, in particular at the end of, the movement of the needle guard sleeve (9) from the proximal end position into the needle-guarding position, the locking means (50) or second locking means (50b) can be moved past the second stop (53), wherein the locking means (50) or second locking means (50b) springs into a position in front of the second stop (53).

8. Injection device according to claim 2, **characterized in that**, when the activation mechanism (13) is in the non-activated position, the first locking means (50a) is positioned in the first position distally and in alignment with the first stop (52), and that by rotation of the activation mechanism (13) the first stop (52) can be rotated out of alignment with the first locking means (50a), and the second locking means (50b) can be deflected and tensed by a second transmission means (51b) into the second position, wherein the first locking means (50a) can be moved in the proximal direction past the first stop (52) and, during, in particular at the end of, the movement of the needle guard sleeve (9) from its proximal end position into its needle-guarding position, the second locking means (50b) can be moved past the second stop (53), wherein the second locking means (50b) springs into a position in front of the second stop.

9. Injection device according to one of the preceding claims, **characterized in that** when the needle guard sleeve (9) is in its needle-guarding position, it projects farther from the housing (1) than it does in its initial position or/and **in that** the first stop (52) is positioned in an axial position different from the axial position of the second stop (53) by reference to the longitudinal axis (L) of the injection device, in particular is disposed proximally of the second stop (53).

10. Injection device according to one of the preceding claims, **characterized in that** the activation mechanism (13) comprises a rotating button which is preferably disposed at the proximal end of the injection device.

11. Injection device according to one of the preceding claims, **characterized in that** the needle guard (9) simultaneously is a trigger mechanism, which can be moved into the proximal end position in order to trigger discharging of the product, and an energy-storing means (6) releases energy in order to discharge the product.

12. Injection device according to one of the preceding claims, **characterized in that** an injection needle (4) is stationary and/or fixed relative to the housing (1) and extends beyond the distal end of the housing (1), wherein the needle guard (9) surrounds the needle tip when in its initial position and can be moved from the initial position into the proximal end position when the activation mechanism (13) is in its activated position, as a result of which the needle (4) projects out from the distal end of the needle guard (9).

13. Injection device according to one of claims 1 to 11, **characterized in that** in order to perform a penetration by the needle, the injection needle (4) can be moved in the distal direction relative to the housing (1) out of a proximal position in which the needle tip is disposed inside the injection device, as a result of which the injection needle (4) projects out from the distal end of the injection device .

## Patentansprüche

1. Injektionsgerät mit einem Mechanismus zur automatischen Entleerung eines Produkts über eine Injektionsnadel (4), welches weiter umfasst:
a) ein Gehäuse (1),
b) einen Nadelschutz (9) am distalen Ende des Injektionsgeräts, worin der Nadelschutz (9) von seiner Anfangsposition in eine proximale Endposition in das Gehäuse (1) bewegt werden kann, und von der proximalen Endposition in eine Nadel-Schutz- Position, in der der Nadelschutz (9) die Injektionsnadel (4), die von einem distalen Ende des Gehäuses (1) vorsteht, umgibt,
c) einen Aktivierungsmechanismus (13), der von einer nicht-aktivierten Position in eine aktivierte Position bewegt werden kann, worin wenn der Aktivierungsmechanismus (13) in der nicht-aktivierten Position ist, der Nadelschutz (9) nicht in die proximale Endposition bewegt werden kann, und wenn der Aktivierungsmechanismus (13) in der aktivierten Position ist, der Nadelschutz (9) in die proximale Endposition bewegt werden kann, **gekennzeichnet durch**
d) ein federnd angeordnetes Sperrmittel (50) oder zweites Sperrmittel (50b) zum Blockieren des Nadelschutzes (9) in der Nadelschutz-Position, und ein Übertragungsmittel (51), das das Sperrmittel (50) oder das zweite Sperrmittel (50b) spannt und während der Bewegung des Aktivierungsmechanismus (13) in die aktivierte Position in eine zweite Position umlenkt.

2. Injektionsgerät nach Anspruch 1, **dadurch gekennzeichnet, dass** das Sperrmittel (50) oder ein erstes Sperrmittel (50a) in einer ersten Position vor einem ersten Stopp (52) angeordnet ist, wobei als ein Ergebnis davon die Bewegung des Nadelschutzes (9) von der Anfangsposition in die proximale Richtung oder in die proximale Endposition blockiert ist.

3. Injektionsgerät nach einem der vorstehenden Ansprüche, **dadurch gekennzeichnet, dass** das Sperrmittel (50) oder das zweite Sperrmittel (50b) vor einem zweiten Stopp (53) angeordnet ist, wenn der Nadelschutz (9) in der Nadelschutz-Position ist, wobei als ein Ergebnis die Bewegung des Nadelschutzes (9) von der Nadelschutz-Position in die proximale Richtung oder in die proximale Endposition blockiert ist.

4. Injektionsgerät nach einem der vorstehenden Ansprüche, **dadurch gekennzeichnet, dass** das Übertragungsmittel (51) angepasst ist, dass es das Sperrmittel (50) oder das zweite Sperrmittel (50b) in der Umfangsrichtung oder transversal zu der Umfangsrichtung, insbesondere in der Umfangsrichtung, aus seiner ersten Position bewegt, wenn der Aktivierungsmechanismus (13) in die aktivierte Position bewegt wird.

5. Injektionsgerät nach einem der vorstehenden Ansprüche, **dadurch gekennzeichnet, dass** der Aktivierungsmechanismus (13) betriebsbereit mit dem Übertragungsmittel (51) verbunden ist, so dass eine Bewegung, insbesondere eine Drehung, des Aktivierungsmechanismus (13), eine Bewegung, insbesondere eine Drehung, des Übertragungsmittels (51) bewirkt, und dass der Nadelschutz (9) betriebsbereit mit dem mindestens einen Sperrmittel (50; 50a, 50b) verbunden ist, so dass eine Bewegung, insbesondere ein Längs- Bewegung, des Nadelschutzes (9) eine Bewegung, insbesondere eine Längs-Bewegung, des mindestens einen Sperrmittels (50; 50a, 50b) bewirkt.

6. Injektionsgeräts nach einem der Ansprüche 1 bis 4, **dadurch gekennzeichnet, dass** der Aktivierungsmechanismus (13) betriebsbereit mit dem mindestens einen Sperrmittel (50; 50a, 50b) verbunden ist, so dass eine Bewegung, insbesondere eine Drehung, des Aktivierungsmechanismus (13) eine Bewegung, insbesondere eine Drehung, des mindestens einen Sperrmittels (50; 50a, 50b) bewirkt, und dass der Nadelschutz (9) betriebsbereit mit dem mindestens einen Übertragungsmittel (51; 51a, 51b) verbunden ist, so dass eine Bewegung, insbesondere eine Längs-Bewegung des Nadelschutzes (9) eine Bewegung, insbesondere eine Längs-Bewegung des mindestens einen Übertragungsmittels (51; 51a, 51b) bewirkt.

7. Injektionsgerät nach einem der vorstehenden Ansprüche, **dadurch gekennzeichnet, dass** das Sperrmittel (50) oder das zweite Sperrmittel (50b) durch Drehung des Aktivierungsmechanismus (13) umgelenkt und durch das Übertragungsmittel (51) in die zweite Position gespannt werden kann, worin der Nadelschutz (9) in die proximale Endposition bewegt werden kann und das Sperrmittel (50) oder das zweite Sperrmittel (50b) in die proximale Richtung bewegt werden kann, und worin während insbesondere am Ende der Bewegung der Nadelschutz-Umhüllung (9) von der proximalen Endposition in die Nadelschutz-Position, das Sperrmittel (50) oder das zweite Sperrmittel (50b) über den zweiten Stopp (53) bewegt werden kann, worin das Sperrmittel (50) oder das zweite Sperrmittel (50b) in eine Position vor dem zweiten Stopp (53) springt.

8. Injektionsgerät nach Anspruch 2, **dadurch gekennzeichnet, dass**, wenn der Aktivierungsmechanismus (13) in der nicht-aktivierten Position ist, das erste Sperrmittel (50a) in der ersten Position distal und ausgerichtet mit dem ersten Stopp (52) angeordnet ist, und dass durch Drehung des Aktivierungsmechanismus (13) der erste Stopp (52) aus der Ausrichtung mit dem ersten Sperrmittel (50a) gedreht werden kann, und das zweite Sperrmittel (50b) durch ein zweites Übertragungsmittel (51b) in die zweite Position umgelenkt und gespannt werden kann, worin das erste Sperrmittel (50a) in die proximale Richtung am ersten Stopp (52) vorbei bewegt werden kann und, während, insbesondere am Ende davon, der Bewegung der Nadelschutz-Umhüllung (9) von deren proximaler Endposition in deren Nadelschutz-Position, das zweite Sperrmittel (50b) am zweiten Stopp (53) vorbei bewegt werden kann, worin das zweite Sperrmittel (50b) in eine Position vor dem zweite Stopp springt.

9. Injektionsgerät nach einem der vorstehenden Ansprüche, **dadurch gekennzeichnet, dass** wenn die Nadelschutz-Umhüllung (9) in deren Nadelschutz-Position ist, sie weiter von dem Gehäuse (1) vorsteht als in deren Ursprungs-Position oder/und dass der erste Stopp (52) in einer axialen Position angeordnet ist, die verschieden ist von der axialen Position des zweiten Stopps (53) in Bezug zu der Längsachse (L) des Injektionsgeräts, insbesondere proximal des zweiten Stopps (53) angeordnet ist.

10. Injektionsgerät nach einem der vorstehenden Ansprüche, **dadurch gekennzeichnet, dass** der Aktivierungsmechanismus (13) einen Drehknopf umfasst, der vorzugsweise an dem proximalen Ende des Injektionsgeräts vorgesehen ist.

11. Injektionsgerät nach einem der vorstehenden Ansprüche, **dadurch gekennzeichnet, dass** der Nadelschutz (9) gleichzeitig ein Auslösemechanismus ist, der in die proximale Endposition bewegt werden kann, um ein Entleeren des Produkts auszulösen, worin ein EnergieSpeichermittel (6) Energie freisetzt, um das Produkt zu entleeren.

12. Injektionsgerät nach einem der vorstehenden Ansprüche, **dadurch gekennzeichnet, dass** eine Injektionsnadel (4) relativ zu dem Gehäuse (1) stationär und/oder fixiert ist und sich über das distale Ende des Gehäuses (1) erstreckt, worin der Nadelschutz (9) die Nadelspitze umgibt, wenn er in seiner Anfangsposition ist und von der Anfangsposition in die proximale Endposition bewegt werden kann, wenn der Aktivierungsmechanismus (13) in seiner aktivierten Position ist, wobei als ein Ergebnis davon die Nadel (4) aus dem distalen Ende des Nadelschutzes (9) vorsteht.

13. Injektionsgerät nach einem der Ansprüche 1 bis 11, **dadurch gekennzeichnet, dass** zur Durchführung einer Penetration durch die Nadel, die Injektionsnadel (4) in der distalen Richtung relativ zu dem Gehäuse (1) aus einer proximalen Position, in der die Nadelspitze in dem Injektionsgerät vorgesehen ist, bewegt werden kann, wobei als ein Ergebnis davon die Injektionsnadel (4) von dem distalen Ende des Injektionsgeräts vorsteht.

## Revendications

1. Dispositif d'injection ayant un mécanisme de décharge automatique d'un produit à travers une aiguille d'injection (4), comprenant en outre :
a) un logement (1),
b) un protecteur d'aiguille (9) au niveau de l'extrémité distale du dispositif d'injection, lequel protecteur d'aiguille (9) peut être déplacé de sa position initiale dans une position d'extrémité proximale dans le logement (1) et de la position d'extrémité proximale dans une position de protection d'aiguille dans laquelle le protecteur d'aiguille (9) entoure l'aiguille d'injection (4) faisant saillie vers l'extérieur à partir d'une extrémité distale du logement (1),
c) un mécanisme d'activation (13) qui peut être déplacé d'une position non activée dans une position activée, et lorsque le mécanisme d'activation (13) est dans la position non activée, le protecteur d'aiguille (9) est bloqué dans sa capacité à être déplacé dans la position d'extrémité proximale et lorsque le mécanisme d'activation (13) est dans la position activée, le protecteur d'aiguille (9) peut être déplacé dans la position d'extrémité proximale,
**caractérisé par**
d) un moyen de verrouillage agencé de manière résiliente (50) ou un deuxième moyen de verrouillage (50b) pour bloquer le protecteur d'aiguille (9) dans la position de protection d'aiguille et un moyen de transmission (51) qui tend et dévie le mécanisme de verrouillage (50) ou le deuxième mécanisme de verrouillage (50b) dans une deuxième position durant le mouvement du mécanisme d'activation (13) dans la position activée.

2. Dispositif d'injection selon la revendication 1, **caractérisé en ce que** le mécanisme de verrouillage (50) ou un premier moyen de verrouillage (50a) est positionné dans une première position devant un premier arrêt (52), en résultat de quoi le mouvement du protecteur d'aiguille (9) de la position initiale dans la direction proximale ou dans la position d'extrémité proximale est bloqué.

3. Dispositif d'injection selon l'une quelconque des revendications précédentes, **caractérisé en ce que** le moyen de verrouillage (50) ou le deuxième moyen de verrouillage (50b) est positionné devant un deuxième arrêt (53) lorsque le protecteur d'aiguille (9) est dans la position de protection d'aiguille, en résultat de quoi le mouvement du protecteur d'aiguille (9) de la position de protection d'aiguille dans la direction proximale ou dans la position d'extrémité proximale est bloqué.

4. Dispositif d'injection selon l'une quelconque des revendications précédentes, **caractérisé en ce que** le moyen de transmission (51) est adapté de manière à ce qu'il déplace le moyen de verrouillage (50) ou le deuxième moyen de verrouillage (50b) dans la direction radiale ou transversalement à la direction radiale, en particulier dans la direction circonférentielle, hors de sa première position lorsque le mécanisme d'activation (13) est déplacé dans la position activée.

5. Dispositif d'injection selon l'une quelconque des revendications précédentes, **caractérisé en ce que** le mécanisme d'activation (13) est fonctionnellement raccordé au moyen de transmission (51) de manière à ce qu'un mouvement, en particulier une rotation, du mécanisme d'activation (13), entraîne un mouvement, en particulier une rotation, du moyen de transmission (51), et **en ce que** le protecteur d'aiguille (9) est fonctionnellement raccordé à l'au moins un moyen de verrouillage (50 ; 50a, 50b) de manière à ce qu'un mouvement, en particulier un mouvement longitudinal, du protecteur d'aiguille (9) entraîne un mouvement, en particulier un mouvement longitudinal, de l'au moins un moyen de verrouillage (50 ; 50a, 50b).

6. Dispositif d'injection selon l'une quelconque des revendications 1 à 4, **caractérisé en ce que** le mécanisme d'activation (13) est fonctionnellement raccordé à l'au moins un moyen de verrouillage (50 ; 50a, 50b) de manière à ce qu'un mouvement, en particulier une rotation, du mécanisme d'activation (13) entraîne un mouvement, en particulier une rotation, de l'au moins un moyen de verrouillage (50 ; 50a, 50b), et le protecteur d'aiguille (9) est fonctionnellement raccordé à l'au moins un moyen de transmission (51 ; 51a, 51b) de manière à ce qu'un mouvement, en particulier un mouvement longitudinal du protecteur d'aiguille (9), entraîne un mouvement, en particulier un mouvement longitudinal, de l'au moins un moyen de transmission (51 ; 51a, 51b).

7. Dispositif d'injection selon l'une quelconque des revendications précédentes, **caractérisé en ce que**, le moyen de verrouillage (50) ou le deuxième moyen de verrouillage (50b) peut, par rotation du mécanisme d'activation (13), être dévié et tendu par le moyen de transmission (51) dans la deuxième position, dans lequel le protecteur d'aiguille (9) peut être déplacé dans la position d'extrémité proximale et le moyen de verrouillage (50) ou deuxième moyen de verrouillage (50b) peut être déplacé dans la direction proximale, et dans lequel durant le, en particulier à la fin du, mouvement du manchon protecteur d'aguille (9) de la position d'extrémité proximale dans la position de protection d'aiguille, le moyen de verrouillage (50) ou le deuxième moyen de verrouillage (50b) peut être déplacé au-delà du deuxième arrêt (53), dans lequel le moyen de verrouillage (50) ou le deuxième moyen de verrouillage (50b) s'encliquette dans une position devant le deuxième arrêt (53).

8. Dispositif d'injection selon la revendication 2, **caractérisé en ce que**, lorsque le mécanisme d'activation (13) est dans la position non activée, le premier moyen de verrouillage (50a) est positionné dans la première position distalement et en alignement avec le premier arrêt (52), et **en ce que** par rotation du mécanisme d'activation (13) le premier arrêt (52) peut être mis en rotation hors d'alignement avec le premier moyen de verrouillage (50a), et le deuxième moyen de verrouillage (50b) peut être dévié et tendu par un deuxième moyen de transmission (51b) dans la deuxième position, dans lequel le premier moyen de verrouillage (50a) peut être déplacé dans la direction proximale au-delà du premier arrêt (52) et, durant le, en particulier à la fin du, mouvement du manchon protecteur d'aiguille (9) de sa position d'extrémité proximale dans sa position de protection d'aiguille, le deuxième moyen de verrouillage (50b) peut être déplacé au-delà du deuxième arrêt (53), dans lequel le deuxième moyen de verrouillage (50b) s'encliquette dans une position devant le deuxième arrêt.

9. Dispositif d'injection selon l'une quelconque des revendications précédentes, **caractérisé en ce que** lorsque le manchon protecteur d'aiguille (9) est dans sa position de protection d'aiguille, il fait saillie plus loin du logement (1) qu'il ne le fait dans sa position initiale et/ou **en ce que** le premier arrêt (52) est positionné dans une position axiale différente de la position axiale du deuxième arrêt (53) par référence à l'axe longitudinal (L) du dispositif d'injection, en particulier est disposé à proximité du deuxième arrêt (53).

10. Dispositif d'injection selon l'une quelconque des revendications précédentes, **caractérisé en ce que** le mécanisme d'activation (13) comprend un bouton de rotation qui est de préférence disposé au niveau de l'extrémité proximale du dispositif d'injection.

11. Dispositif d'injection selon l'une quelconque des revendications précédentes, **caractérisé en ce que** le protecteur d'aiguille (9) est simultanément un mécanisme de déclenchement, qui peut être déplacé dans la position d'extrémité proximale de manière à déclencher le déchargement du produit, et un moyen de stockage d'énergie (6) libère de l'énergie afin de décharger le produit.

12. Dispositif d'injection selon l'une quelconque des revendications précédentes **caractérisé en ce qu'**une aiguille d'injection (4) est stationnaire et/ou fixe par rapport au logement (1) et s'étend au-delà de l'extrémité distale du logement (1), dans lequel le protecteur d'aiguille (9) entoure la pointe d'aiguille lorsqu'elle est dans sa position initiale et peut être déplacée de la position initiale dans la position d'extrémité proximale lorsque le mécanisme d'activation (13) est dans sa position activée, en résultat de quoi l'aiguille (4) fait saillie vers l'extérieur à partir de l'extrémité distale du protecteur d'aiguille (9).

13. Dispositif d'injection selon l'une quelconque des revendications 1 à 11, **caractérisé en ce qu'**afin de réaliser une pénétration par l'aiguille, l'aiguille d'injection (4) peut être déplacée dans la direction distale par rapport au logement (1) hors d'une position proximale dans laquelle la pointe d'aiguille est disposée à l'intérieur du dispositif d'injection, en résultat de quoi l'aiguille d'injection (4) fait saillie hors de l'extrémité distale du dispositif d'injection.
